# EUROPEAN PATENT SPECIFICATION

(11) **EP 3 256 474 B1**
(45) Date of publication and mention of the grant of the patent: **25.09.2019**
(21) Application number: 16706789.1
(22) Date of filing: 09.02.2016
(51) Int. Cl.: C07D 487/04, A61K 31/519

(54) **IBRUTINIB SULPHATE SALT**
IBRUTINIB-SULFATSALZ
SEL DE SULFATE IBRUTINIB

(30) Priority: 09.02.2015 CZ 20150084
(43) Date of publication of application: 20.12.2017
(73) Proprietor: Zentiva K.S., 102 37 Praha 10 (CZ)
(72) Inventor: ZVATORA, Pavel, 798 03 Plumlov (CZ); DAMMER, Ondrej, 253 01 Hostivice (CZ); TKADLECOVA, Marcela, 163 00 Praha 6 (CZ); KREJCIK, Lukas, 190 17 Praha-Vinor (CZ); BERANEK, Josef, 160 00 Praha 6 (CZ)
(74) Representative: Jirotkova, Ivana
(86) International application number: PCT/CZ2016/000014
(87) International publication number: WO 2016/127960

(56) References cited:
- WO-A1-2013/184572
- WO-A2-2008/039218

## Description

### Technical Field

The invention relates to a novel solid form of 1-[(3*R*)-3-[4-amino-3-(4-phenoxyphenyl)-1H-pyrazolo[3,4-d]pyrimidin-1-yl]piperidin-1-yl]prop-2-en-1-one of formula (I), 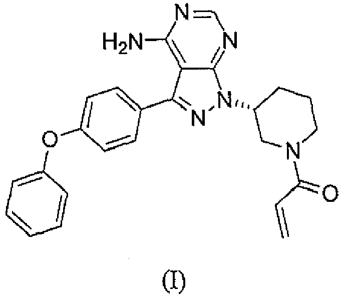 known as ibrutinib, and methods of its preparation.

### Background Art

1-[(3*R*)-3-[4-Amino-3-(4-phenoxyphenyl)-1H-pyrazolo[3,4-d]pyrimidin-1-yl]piperidin-1-yl]prop-2-en-1-one, which is known as ibrutinib (CAS no. 936563-96-1), belongs to the group of kinase inhibitors that can be used for the treatment of lymphomas. Under the trade name Imbruvica, Ibrutinib was approved by The Food and Drug Administration (FDA) for the treatment of centrocytic lymphoma.

Preparation of this molecule and its isolation with the use of chromatography was described in the document WO2008/039218. Preparation of crystalline forms of ibrutinib in a solvated or non-solvated form was described in the documents WO2013/184572 and CN 103694241. Use of ibrutinib in the treatment of lymphomas together with other pharmaceutically active ingredients was described in the document WO2013/113841 and the document WO2013/155347, which also mentions pharmaceutically acceptable salts of this drug without any further description or example of production. Besides preparation methods, the document WO2013/184572 deals with pharmaceutical compositions of crystalline and solvated forms of ibrutinib for oral administration and mentions pharmaceutically acceptable salts of this drug without any further description or example of production. Further use of ibrutinib for the treatment of cancer, inflammatory and autoimmune diseases, together with a description of pharmaceutical compositions, with mentioning its pharmaceutically acceptable salts without their further description or example of production is mentioned in the patent WO2014/004707.

### Disclosure of Invention

The invention provides a novel process for preparing a salt of ibrutinib of formula I with sulphuric acid in a solid form.

The process comprises mixing 1-[(3R)-3-[4-amino-3-(4-phenoxyphenyl)-1H-pyrazolo[3,4-d]pyrimidin-1-yl]piperidin-1-yl]prop-2-en-1-one with sulphuric acid and a solvent, wherein the solvent is selected either
a) from a C1 to C4 alcohol, from which ibrutinib sulphate in a crystalline form, which exhibits a peak with the maximum at 123°C in the DSC record is obtained, whereupon crystallization follows, or
b) from C3 to C7 ketones, from which amorphous ibrutinib sulphate is precipitated by addition of an antisolvent selected from methyl *tert*-butyl ether.

The prepared new solid form has suitable physical-chemical characteristics for use in the pharmaceutical industry and in formulation of new dosage forms. The amorphous form of ibrutinib sulphate exhibits an approximately three times higher dissolution rate than its crystalline basic form (form A).

### Brief Description of Drawings

- Figure 1.: X-ray powder pattern of ibrutinib sulphate (according to Example 2)
- Figure 2.: ¹H NMR of ibrutinib sulphate (according to Example 2)
- Figure 3.: DSC record of ibrutinib sulphate (according to Example 2)
- Figure 4: IR spectrum of ibrutinib sulphate (according to Example 2)
- Figure 5.: X-ray powder pattern of ibrutinib sulphate (according to Example 3)
- Figure 6.: ¹H NMR of ibrutinib sulphate (according to Example 3)
- Figure 7.: DSC record of ibrutinib sulphate (according to Example 3)
- Figure 8.: IR spectrum of ibrutinib sulphate (according to Example 3)
- Figure 9.: X-ray powder pattern of ibrutinib sulphate (according to Example 4)

### Detailed description of the invention

Although preparation of a salt by a reaction of an acid and base is a well-known method, it is always a problem to obtain the required salts in the solid phase and in a purity corresponding to the demands for their pharmaceutical use. Biological availability greatly depends on whether a crystalline or amorphous product is obtained. An amorphous product is usually more readily soluble, it cannot often be obtained in the required quality and it is also often unstable. Conversely, compared to the amorphous form, a crystalline product is often stable, its required purity is easier to achieve and it dissolves more slowly. Mixtures of the amorphous and crystalline solid phase may represent a solution to the problem.

The process of this invention provides the salt of ibrutinib in the solid phase in an amorphous form or in a mixture of an amorphous and crystalline form.

The process according to the invention provides a novel solid form of ibrutinib with sulphuric acid, which can be prepared in adequate yields with high chemical purity in a crystalline form, in an amorphous form, or in a mixture of amorphous and crystalline forms.

The prepared novel solid form of ibrutinib may have various internal arrangements (polymorphism) with different physical-chemical properties depending on the conditions of its preparation. Therefore, the disclosure relates to a crystalline or amorphous form of ibrutinib in any ratio.

These novel solid forms are suitable for preparation of ibrutinib with a high chemical and optical purity.

The most commonly used solvents are acetone, ethanol, isopropanol, acetonitrile, tetrahydrofuran or their mixtures.

The resulting product is precipitated or crystallized, typically at temperatures in the range of -30°C to the boiling point of the solvent.

The free base of ibrutinib was prepared in accordance with the procedure mentioned in the patent (WO2008/039218).

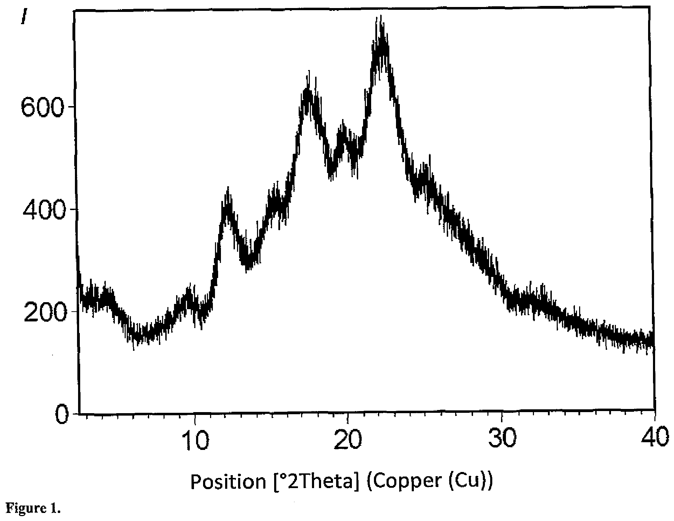

The X-ray powder pattern of a partly crystalline form of ibrutinib sulphate (prepared according to Example 2) is shown in Fig. 1.

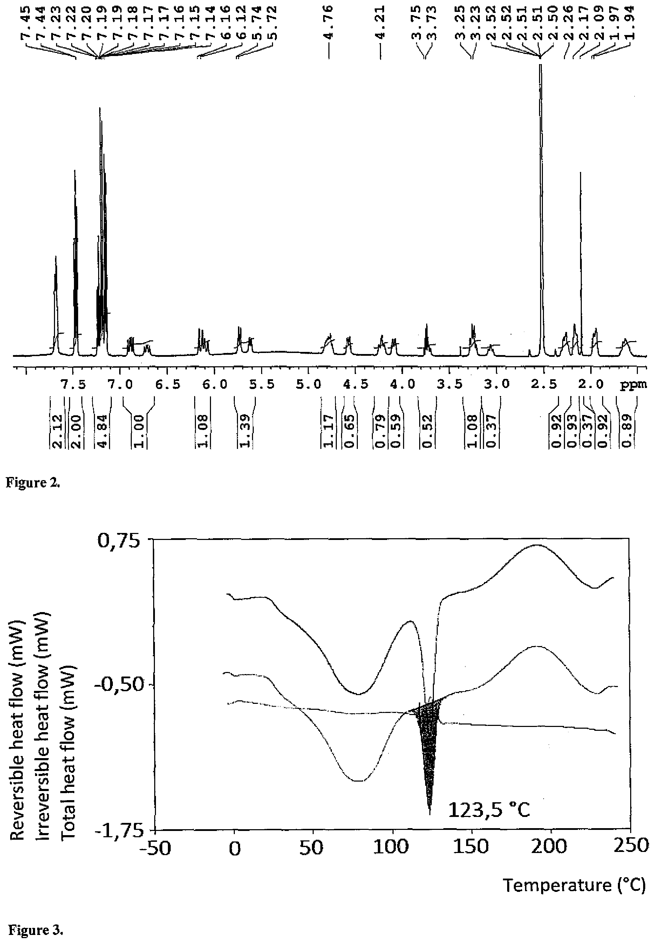

Figure 2 shows an example of the ¹H NMR spectrum of the prepared ibrutinib sulphate (according to Example 2).

A DSC record of ibrutinib sulphate (prepared according to Example 2) is shown in Fig. 3. According to this example the melting point of ibrutinib sulphate is 123°C.

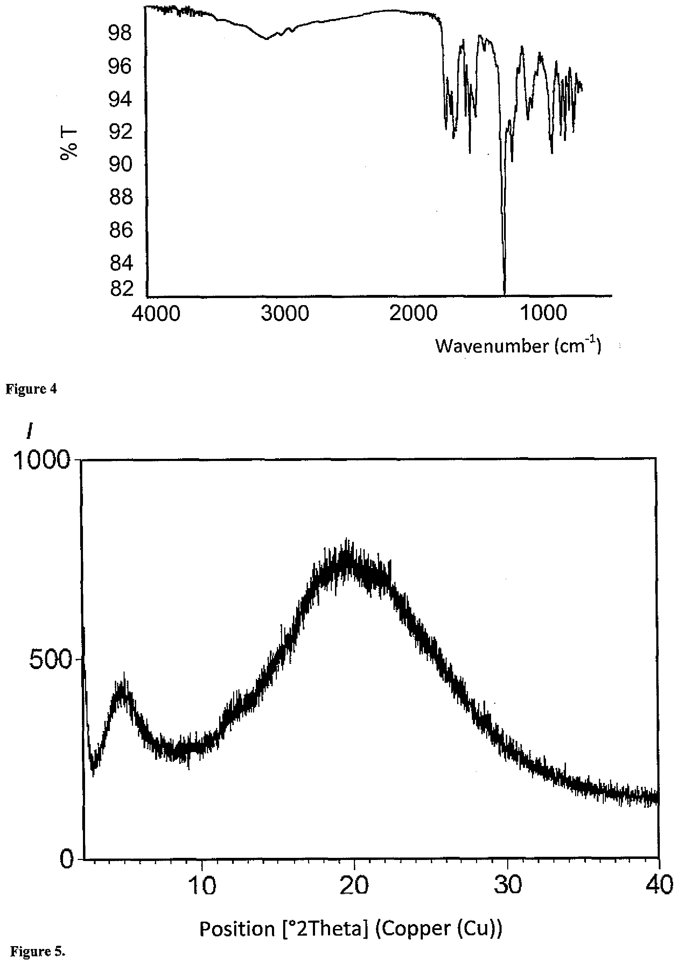

An infrared spectrum of ibrutinib sulphate (prepared according to Example 2) is shown in Fig. 4. The molar ratio of ibrutinib:sulphuric acid can be in the range of 10:1 to 1:3, preferably 1:1, 2: 1 and 1:2.

The X-ray powder pattern of an amorphous form of ibrutinib sulphate (prepared according to Example 3) is shown in Fig. 5.

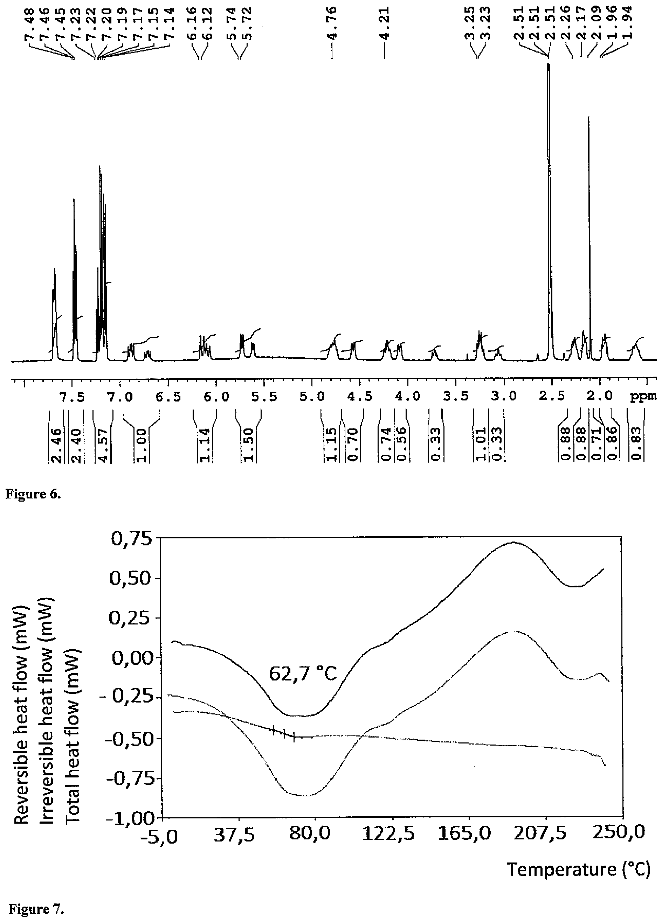

Figure 6 shows an example of the ¹H NMR spectrum of the prepared ibrutinib sulphate (according to Example 3).

A DSC record of ibrutinib sulphate (prepared according to Example 3) is shown in Fig. 7. According to this example the glass transition temperature of the amorphous form of ibrutinib sulphate is 63°C.

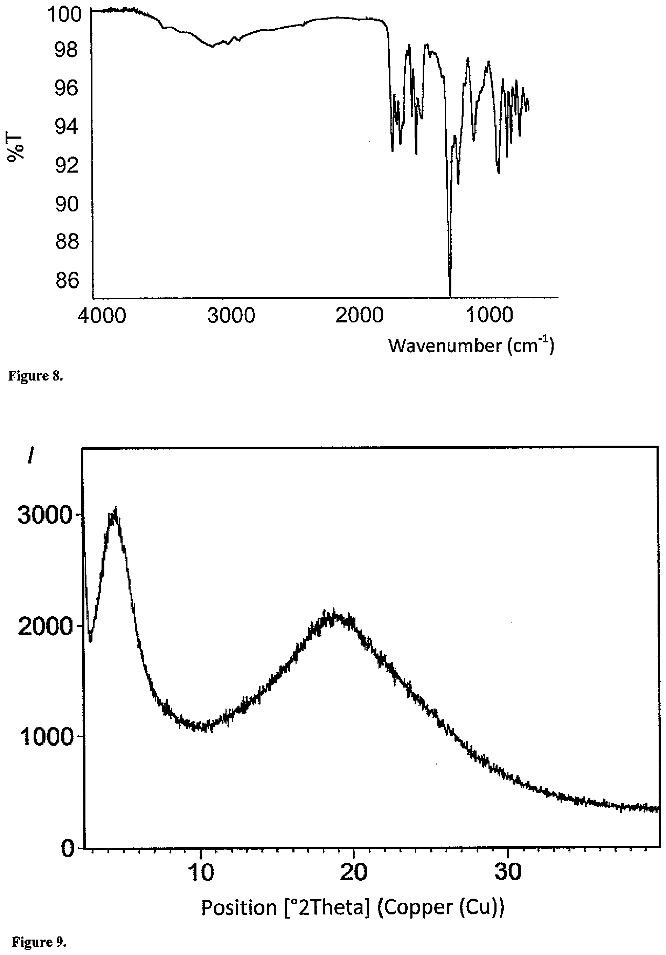

The infrared spectrum of ibrutinib sulphate is shown in Fig. 8. The molar ratio of ibrutinib:sulphuric acid can be in the range of 10:1 to 1:3, preferably 1:1, 2:1 and 1:2.

The X-ray powder pattern of an amorphous form of ibrutinib sulphate (prepared according to Example 4) is shown in Fig. 9.

Advantageous characteristics of the novel salt of ibrutinib can be documented by increased solubility.

The dissolution rate of the amorphous form of ibrutinib sulphate (prepared according to Example 3) in a pH 2 solution is 0.043 mg·min⁻¹·cm⁻², while, under identical conditions, solubility of the crystalline base of ibrutinib (form A) is 0.017 mg·min⁻¹·cm⁻².

The invention is clarified in a more detailed way using the working examples below. These examples, which illustrate the preparation of the novel solid forms of ibrutinib only have an illustrative character and do not restrict the scope of the invention in any respect.

### Experimental part

### X-ray powder diffraction

The diffraction patterns were obtained using an X'PERT PRO MPD PANalytical powder diffractometer, used radiation CuKα (λ=1.542 Å), excitation voltage: 45 kV, anode current: 40 mA, measured range: 2 - 40° 2θ, increment: 0.01° 2θ at the dwell time at a reflection of 0.5 s; the measurement was carried out with a flat sample with the area/thickness of 10/0.5 mm. For the correction of the primary array 0.02 rad Soller slits, a 10mm mask and a 1/4° fixed anti-dispersion slit were used. The irradiated area of the sample is 10 mm, programmable divergence slits were used. For the correction of the secondary array 0.02 rad Soller slits and a 5.0 anti-dispersion slit were used.

### Infrared spectroscopy

ATR (Ge - single reflection) infrared spectra of the powder samples were measured with an infrared spectrometer (Nicolet Nexus, Thermo, USA), equipped with a DTGS KBr detector, in the measurement range of 600-4000 cm⁻¹ and the spectral resolution of 4.0 cm⁻¹. The data were obtained at 64 spectrum accumulations. The OMNIC 6.2 software was used to process the spectra.

### Differential Scanning Calorimetry (DSC)

The records of the novel solid forms of ibrutinib sulphate were measured with the Discovery DSC device made by TA Instruments. The sample charge in a standard Al pot (40 µL) was between 4-5 and 5 mg and the heating rate was 5°C/min. The temperature program that was used consists of 1 min of stabilization at the temperature of 0°C and then of heating up to 220°C at the heating rate of 5°C/min (Amplitude = 0.8°C and Period = 60 s). 5.0 N₂ at the flow rate of 50 ml/min was used ss the carrier gas.

### ¹H NMR

For the structural characterization ¹H NMR spectroscopy at 250 MHz by Bruker Avance 250 was used. Deuterated D6-dimethyl sulfoxide was used as the solvent and the measurements were carried out at the temperature of 303 K. Trimethylsilane (TMS) was used as the internal reference with 0.00 ppm.

### Examples

### Example 1

The free base of ibrutinib was prepared in accordance with the procedure mentioned in the patent (WO2008/039218).

### Example 2

### Preparation of ibrutinib sulphate in ethanol

1-[(3*R*)-3-[4-Amino-34-phenoxyphenyl)-1H-pyrazolo[3,4-d]pyrimidin-1-yl]piperidin-1-yl]prop-2-en-1-one (ibrutinib) in the amount of 100 mg (2.27·10⁻⁴ mol) was dissolved in 20 ml of ethanol. 23.2 mg (2.27·10⁻⁴ mol) of sulphuric acid (96%) was added to this solution. The mixture was left under stirring at the room temperature for 3 hours. The resulting solution was slowly concentrated by partial evaporation of the solvent (final volume 5 ml) in a vacuum evaporator at the room temperature. The solution was kept in a refrigerator overnight. The separated solid fraction was collected by filtration and dried at 30 to 50°C in a vacuum drier for 16 hours. Yield 83 mg (67%). HPLC purity 99%. Melting point 123°C (DSC). XRPD: Fig. 1.

### Example 3

### Preparation of ibrutinib sulphate in acetone

1-[(3*R*)-3-[4-Amino-3-(4-phenoxyphenyl)-1H-pyrazolo[3,4-d]pyrimidin-1-yl]piperidin-1-yl]prop-2-en-1-one (ibrutinib) in the amount of 230 mg (5.22·10⁻⁴ mol) was dissolved in 9 ml of acetone. 53.3 mg (5.22·10⁻⁴ mol) of sulphuric acid (96%) was added to this solution. The mixture was left under stirring at the room temperature for 3 hours. The resulting solution was evaporated in a vacuum evaporator at 4°C. Yield 280.5 mg (99%). HPLC purity 99%. Glass transition temperature 63°C (DSC). XRPD: Fig. 5.

### Example 4

### Preparation of ibrutinib sulphate in acetone (by precipitation)

1-[(3*R*)-3-[4-Amino-3-(4-phenoxyphenyl)-1H-pyrazolo[3,4-d]pyrimidin-1-yl]piperidin-1-yl]prop-2-en-1-one (ibrutinib) in the amount of 300 mg (6.81·10⁻⁴ mol) was dissolved in 12.2 ml of acetone. 71 mg (6.95·10⁻⁴ mol) of sulphuric acid (96%) was added to this solution. The mixture was left under stirring at the room temperature for 3 hours. Then, this solution was stirred in an ice bath at 0°C and 48 ml of methyl *tert*-butyl ether was added under intensive stirring. The resulting precipitate was left under stirring for 30 min and then filtered. Yield 363.6 mg (98%). HPLC purity 99%. HPLC purity 99%. XRPD: Fig. 9.

## Claims

1. A process for preparing a salt of ibrutinib of formula I with sulphuric acid in a solid form. 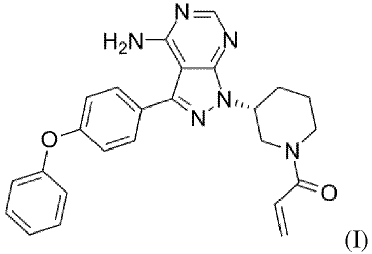 **characterized in that** 1-[(3R)-3-[4-amino-3-(4-phenoxyphenyl)-1H-pyrazolo[3,4-d]pyrimidin-1-yl]piperidin-1-yl]prop-2-en-1-one is mixed with sulphuric acid and a solvent, wherein the solvent is selected either
a. from a C1 to C4 alcohol, from which ibrutinib sulphate in a crystalline form, which exhibits a peak with the maximum at 123°C in the DSC record is obtained, whereupon crystallization follows, or
b. from C3 to C7 ketones, from which amorphous ibrutinib sulphate is precipitated by addition of an antisolvent selected from methyl *tert*-butyl ether.

2. The process according to claim 1, **characterized in that** the crystallization is performed from ethanol.

3. The process according to claim 1, **characterized in that** amorphous ibrutinib sulphate is precipitated from acetone.

## Patentansprüche

1. Verfahren zur Herstellung einer festen Form eines Ibrutinib-Salzes der Formel I mit Schwefelsäure 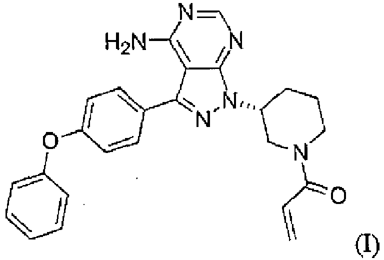 **dadurch gekennzeichnet, dass** 1-[(3R)-3-[4-Amino-3-(4-phenoxyphenyl)-1H-pyrazolo[3,4-d]pyrimidin-1-yl]piperidin-1-yl]prop-2-en-1-on mit Schwefelsäure und einem Lösungsmittel gemischt wird, wobei das Lösungsmittel entweder ausgewählt ist aus
a. einem C1-bis-C4-Alkohol, wobei Ibrutinibsulfat in einer kristallinen Form erhalten wird, welche im DSC-Diagramm einen Peak mit dem Maximum bei 123 °C aufweist, worauf Kristallisation erfolgt, oder aus
b. C3-bis-C7-Ketonen, woraus durch Zugabe eines Fällungsmittels, ausgewählt aus Methyl-*tert*-butylether, amorphes Ibrutinibsulfat ausfällt.

2. Verfahren nach Anspruch 1, **dadurch gekennzeichnet, dass** die Kristallisation aus Ethanol erfolgt.

3. Verfahren nach Anspruch 1, **dadurch gekennzeichnet, dass** das amorphe Ibrutinibsulfat aus Aceton ausgefällt wird.

## Revendications

1. Un procédé de préparation d'un sel d'ibrutinib de formule 1 par réaction avec de l'acide sulfurique sous forme solide : 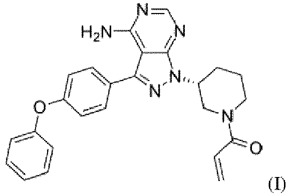 **caractérisé en ce que** on mélange 1-[(3R)-3-[4-amino-3-(4-phénoxyphényl)-1H-pyrazolo[3,4-d]pyrimidin-1-yl]pipéridin-1-yl]prop-2-én-1-one avec de l'acide sulfurique et un solvant, dans lequel le solvant est choisi entre :
a. soit un alcool en C₁ à C₄, dont est obtenu le sulfate d'ibrutinib sous forme cristalline, présentant un pic avec un maximum à 123°C dans l'enregistrement DSC, ce que l'on fait suivre d'une cristallisation, ou
b. soit les cétones en C₃ à C₇, dont par addition d'un antisolvant choisi de l'éther méthylique de *tert*-butyle, précipite le sulfate d'ibrutinib amorphe.

2. Le procédé selon la revendication 1, **caractérisé en ce que** la cristallisation est effectuée à partir de l'éthanol.

3. Le procédé selon la revendication 1, **caractérisé en ce que** le sulfate d'ibrutinib amorphe est précipité à partir de l'acétone.
